## Europäisches Patentamt

## European Patent Office

## Office européen des brevets

(19)

(11) Veröffentlichungsnummer: **0 149 744**
**B1**

(12) **EUROPÄISCHE PATENTSCHRIFT**

(45) Veröffentlichungstag der Patentschrift:
03.02.88

(21) Anmeldenummer: 84113703.7

(22) Anmeldetag: 14.11.84

(51) Int. Cl.⁴: **C 12 P 7/62** //
(C12P7/62, C12R1:05)

(54) Verfahren zur biotechnologischen Herstellung von Poly-D(-)-3-hydroxybuttersäure.

(30) Priorität: 01.12.83 DE 3343576

(43) Veröffentlichungstag der Anmeldung:
31.07.85 Patentblatt 85/31

(45) Bekanntmachung des Hinweises auf die Patenterteilung:
03.02.88 Patentblatt 88/5

(84) Benannte Vertragsstaaten:
AT BE CH DE FR GB IT LI LU NL SE

(56) Entgegenhaltungen:
EP-A-0 046 344

CHEMICAL ABSTRACT, Band 95, Nr. 23, 3.
Dezember 1981, Seite 341, Nr. 21041r, COLUMBUS,
OHIO, (US). K.A. MALIK et al.: "Nitrogen fixytion
by the hydrogen-oxidizing bacterium Alcaligenes
latus".
CHEMICAL ABSTRACTS, Band 91, nr. 3, 20. Juli
1979, Seite 307, Nr. 189420e, COLUMBUS, OHIO,
(US). D. VOLLBRECHT et al.: "Excretion of
metabolites by hydrogen bacteria. IV. Respiration
ratedependent formation of primary metabolites
and of poly-3-hydroxybutanoate.

(73) Patentinhaber: CHEMIE LINZ
AKTIENGESELLSCHAFT, St. Peter- Strasse 25,
A-4020 Linz (AT)

(84) Benannte Vertragsstaaten:
BE CH FR GB IT LI LU NL SE AT

(73) Patentinhaber: Lentia Gesellschaft mit
beschränkter Haftung, Arabellastrasse 4
Postfach 81 05 08, D-8000 München 81 (DE)

(84) Benannte Vertragsstaaten: DE

(72) Erfinder: Lafferty, Robert M., Dr., Dolezalgasse 21,
A-8051 Graz (AT)
Erfinder: Braunegg, Gerhart, Dipl.- Ing. Dr.,
Kurzeggerweg 14, A-8044 Graz (AT)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Es gilt erst als eingelegt, wenn die Einspruchsgebühr entrichtet worden ist (Art. 99(1) Europäisches Patentübereinkommen).

LIBER, STOCKHOLM 1988

0 149 744

**Beschreibung**

Die vorliegende Erfindung betrifft ein Verfahren zur biotechnologischen Herstellung von Poly-D(-)-3-hydroxybuttersäure (PHB) mit einem hohen Ertragskoeffizienten und einer verbesserten Anreicherung von PHB im bakteriellen Zellmaterial.

Es ist seit längerer Zeit bekannt, daß eine Vielzahl von Mikroorganismen prokaryotischer Natur imstande ist, PHB als Speicherstoff für Energie und Kohlenstoff zellintern anzuhäufen. Die aus dem Zellmaterial des Mikroorgsnismus isolierte PHB ist ein thermoplastischer Polyester mit günstigen Physikalischen Eigenschaften, die seine Verwendung für ähnliche Zwecke erwarten lassen, für welche heute beispielsweise Polyäthylen oder Polystyrol zur Verfügung stehen. Gegenüber diesen heute gebräuchlichen Polymeren hat die PHB aber den Vorteil, daß sie auf biotechnologischem Weg zugänglich und auf biologischem Weg auch wieder abbaubar ist.

Es ist auch schon bekannt, daß die aerobe Kultivierung von PHB-speichernden Mikroorganismen durch die spezifische Zusammensetzung des Nährmediums bevorzugt in Richtung von Zellteilung und Wachstum oder in Richtung von zellinterner PHB-Speicherung gesteuert werden kann. Eine Anreicherung von PHB in den Zellen des Mikroorganismus wird bei den bekannten Verfahren dann erreicht, wenn im Nährmedium die Konzentration der Kohlenstoffquelle im Verhältnis zum Angebot an anderen für das Wachstum erforderlichen Nährstoffen, beispielsweise Stickstoff und Phosphor groß ist und die Züchtung des Mikroorganismus beispielsweise unter ammoniumlimitierten Wachstumsbedingungen erfolgt.

Ein auf diesen Erkenntnissen beruhendes zweistufiges Verfahren zur Herstellung von PHB ist z. B. in der EP-A -15 669 beschrieben. Bei diesem Verfahren wird ein Mikroorganismus von der Gattung Methylobacterium organophilium zunächst bei unbeschränkter Versorgung mit Nährstoffen, einschließlich einer vollständigen und ausreichenden Versorgung mit Stickstoff und Phosphor, gezüchtet, bis die Mikroorganismenpopulation eine Konzentration von vorzugsweise 20 bis 25 g Biomasse pro Liter Kulturflüssigkeit erreicht hat, ohne daß in dieser Wachstumsphase eine zellinterne PHB-Speicherung auftritt. Die vollständige Unterbrechung der Stickstoff- und/oder Phosphorversorgung des Kulturmediums in der zweiten Fermentationsstufe bewirkt dann einen Stillstand der Reproduktion und des Bakterienwachstums, wobei erst in dieser Phase zellintern PHB-Speicherung eintritt. Nach den Angaben in der EP-A-15 669 werden bei diesem Verfahren Biomassen mit einem PHB-Gehalt von höchstens 25 bis 47 Gew.-% des Zelltrockengewichts erhalten.

In der EP-A-46 344 ist ein dem gegenüber verbessertes Fermentationsverfahren für die Herstellung von PHB beschrieben, welches auf einer kontinuierlichen aeroben Kultivierung von Mikroorganismen der Species Alcaligenes eutrophus beruht. Nach den dort gemachten Angaben gelingt es bei diesem Verfahren ein kontrolliertes Wachstum der Mikroorganismenpopulation und gleichzeitig eine zellinterne PHB-Anreicherung dadurch zu erzielen, daß im Gegensatz zum Verfahren der EP-A-15 669 die Versorgung mit den für das Wachstum essentiellen Nähr- und Spurenstoffen, insbesonders die Versorgung mit Stickstoff, von Beginn der Kultivierung an beschränkt wird. Auf diese Weise wird zwar eine bessere Ausnützung der Kohlenstoffquelle im Nährmedium und eine verbesserte Speicherung der PHB erreicht, dem Verfahren haftet jedoch der Nachteil an, daß durch die Unterversorgung mit wachstumsessentiellen Nährstoffen sowohl die spezifische Wachstumsgeschwindigkeit als auch die PHB-Produktbildungsgeschwindigkeit deutlich herabgesetzt wird.

Ein weiterer Nachteil bei diesem Verfahren ist darin zu sehen, daß bei der Species Alcaligenes eutrophus der optimale Temperaturbereich für die Fermentierung verhältnismäßig tief liegt. Der Mikroorganismus muß daher unter starker Kühlung bei 30 bis 34°C in einem Temperaturberich gezüchtet werden, in dem sowohl Wachstum als auch PHB-Speicherung langsamer verlaufen, als es bei Mikroorganismen mit einer größeren Thermotoleranz, die bei höher Temperatur kultiviert werden können, der Fall ist.

Aus den genannten Gründen wird daher bei den bekannten Verfahren die Wirtschaftlichkeit der PHB-Herstellung durch die langen Verweilzeiten im Fermenter, die für die Züchtung elner Biomasse mit einer befriedigenden Speichermenge an PHB notwendig sind, beeinträchtigt.

Als Kohlenstoffquelle unter den Kohlehydraten dient beim Verfahren der EP-A-46 344 in erster Linie Fructose und nur für den Fall, daß spezielle vom Stamm Alcaligenes eutrophus H 16 abgeleitete Mutanten verwendet werden, kann auf Glucose als Nährstoffquelle ausgewichen werden. Neben dem Umstand, daß die Züchtung und Auslese der Mutanten aus dem Elternstamm arbeitsaufwendig ist, wird durch den Einsatz dieser Mutanten das Angebot an verwertbaren Nährstoffquellen, die eine wirtschaftliche Herstellung von PHB ermöglichen würden, nicht wesentlich erweitert, da die in großer Menge zur Verfügung stehenden Disaccharide, die als Hauptlieferant für Glucose dienen, beispielsweise die in der Melasse oder Industriellen Zuckerlösungen enthaltene Saccharose, auch für die von Alcaligenes eutrophus H 16 abgeleiteten Mutanten nicht verwertbar sind.

Neben Alcaligenes eutrophus werden in der EP-A-46 344 zwar auch noch andere Species der Gattung Alcaligenes als brauchbar für die PHB-Speicherung erwähnt, belspielsweise Alc. faecalis, Alc. ruhlandil, Alc. latus, Alc. aquamarinus, jedoch werden für keine dieser Species nähere Angaben über Züchtungs- und Anreicherungsbedingungen gemacht und die Durchführung der Erfindung ist in der Beschreibung und in den Beispielen nur mit Stämmen von Alcaligenes eutrophus geoffenbart.

Demgegenüber liegt dieser Erfindung die Aufgabe zugrunde, ein wirtschaftlicheres Verfahren zur biotechnolgischen Herstellung von PHB mit einem hohen Ertragskoeffizienten und einer verbesserten Anreicherung von PHB im Zellmaterial des Mikroorganismus unter Ausnützung von billigeren Kohlenstoffquellen zu schaffen, bei dem die den bekannten Verfahren anhaftenden Nachteile vermlieden werden.

2

Bei der Lösung dieser Aufgabe hat sich nun überraschenderweise gezeigt, daß ein sehr rasches Wachstum der Mikroorgarnismenpopulation mit gleichzeitiger effektiver PHB-Speicherung bei kürzeren Verweilzeiten im Fermenter erzielt werden und die Abhängigkeit des Mikroorganismus von der Natur der vewertbaren Kohlenstoffquelle weitgehend beseitigt werden kann, wenn man Stämme der Species Alcaligenes latus oder deren Mutanten im Temperaturbereich von 36 bis 42°C kultiviert und das Fermentationsverfahren unter Bedingungen durchführt, die bisher bei der fermentativen Herstellung von PHB nicht bekannt waren.

Zu den wesentlichen Merkmalen dieses neuen Produktionsverfahrens für PHB gehört, daß man im Gegensatz zum Stand der Technik durch eine optimale, unbeschränkte Nährstoffversorgung, einschließlich einer vollständigen und ausreichenden Versorgung mit Stickstoff und Phosphor, die Reproduktion und das rasche Wachstum der Mikroorganismenpopulation fördert und dabei überraschenderweise gleichzeitig eine wirksame intrazelluläre PHB-Speicherung erzielt, ohne daß man die Kultur wachstumslimitierenden Bedingungen unterwirft.

Auf diese Weise entstehen innerhalb von kürzeren Verweilzeiten im Fermenter Biomassen mit einer bedeutend besseren Anreicherung an PHB als es dem Stand der Technik entspricht, wodurch die Herstellung und Isolierung der PHB auf wirtschaftlichere Weise, als es bisher möglich war, gelingt.

Gegenstand der vorliegenden Erfindung ist demnach ein Verfahren zur biotechnologischen Herstellung von Poly-D-(-)-3-hydroxybuttersäure durch aerobe Kultivierung eines Mikroorganismus der Gattung Alcaligenes in einem wässerigen Nährmedium, das Quellen für assimilierbaren Kohlenstoff, Stickstoff und Phosphor, sowie das für das Wachstum des Mikroorganismus erforderliche Angebot an Spurennährstoffen enthält, wobei dieses Verfahren dadurch gekennzeichnet ist, daß man einen Stamm von Alcaligenes latus oder eine von diesem Mikroorganismus abgeleitete PHB-speichernde Mutante bei vollständiger und für das Wachstum des Mikroorganismus optimaler Nährstoffversorgung unter unlimitierten Wachstumsbedingungen im Temperaturbereich von 36 bis 42°C bei einem Gelöstsauerstoffgehalt von 25 bis 50 % des Sättigungswertes für Luft und einem pH-Wert von 6.0 bis 7.5 unter sterilen Bedingungen züchtet und aus der dabei erhaltenen Biomasse die PHB auf übliche Weise durch Extraktion gewinnt.

Zur Durchführung des Verfahrens eignen sich alle bekannten Stämme von Alcaligenes latus, beispielsweise die Stämme DSM (Deutsche Sammlung von Mikroorganismen) Nr. 1122, DSM Nr. 1123 oder DSM Nr. 1124.

Die morphologischen Eigenschaften dieser Stämme sind in der Literatur bei Palleroni et al. im Int. Journ. of Systematic Bacteriology 28, 416-428, 1978 beschrieben und in Katalogen von Kultursammlungen, beispielsweise in der American Type Culture Collection (ATCC), verzeichnet. Kulturproben der durch DSM-Nummern identifizierten Mikroorganismen sind von der Deutschen Sammlung für Mikroorganismen in Göttingen der Gesellschaft für biotechnologische Forschung, MBH in Stöckheim, Deutsche Bundesrepublik für den Fachmann frei erhältlich.

Stämme von Alcaligenes latus haben die für eine wirtschaftliche PHB-Produktion vorteilhafte Eigenschaft, daß sie in der Lage sind, eine breite Palette von Kohlenstoffquellen zum Wachstum und zur PHB-Speicherung zu verwerten. Als Kohlenstoffquellen sind beispielsweise zu nennen Kohlehydrate, wie D-Glucose, D-Fructose, Lactose, Maltose, Saccharose, Stärke, Melasse, Betain, Grünsirup, Hydrol, Zellulosehydrolysate; organische Säuren bzw. deren Ester und Salze, wie wasserlösliche Salze der Glukon-, 2-Keto-glukon,-Ameisen-, Essig-, Butter-, Isobutter-, L-Malon-, D-(-)-Wein-, Aconit-, Itacon-, m-Hydroxybenzoe-, p-Hydroxybenzoe-, Gentisin-, Protocatechu- und Mandelsäure; Alkohole wie n-Propanol, Isopropanol 2,3-Butylenglycol Propylenglycol, Glycerin; Aminosäuren, wie β-Allanin, L-Alanin, L-Serin, L-Threonin, L-Leucin, L-Citrullin, L-Ornithin, L-Aminobutyrat, L-Asparat, L-Asparagin, L-Glutamat, L-Prolin, Hippurat, Sarcosin und Creatin; oder auch Amine, wie Butylamin.

Gemäß ihren Eigenschaften als fakultativ chemolithoautotrophe Mikroorganismen sind die Stämme von Alcaligenes latus aber auch noch in der Lage, neben den bisher aufgezählten Kohlenstoffquellen Kohlendioxld zu verwerten, wenn Kohlendioxid in einem Gemisch mit Wasserstoff und Sauerstoff zusammen als einzige Kohlenstoffquelle angeboten wird.

Da die Wirtschaftlichkeit eines biotechnologischen Verfahrens zur Herstellung von PHB sehr wesentlich vom Preis der Kohlenstoffquelle im Nährsubstrat abhängt, ist die Verwertbarkeit einer so großen Anzahl von Verbindungen beim erfindungsgemäßen Fermentierungsverfahren von großem Vorteil, da auf die jeweils billigste Kohlenstoffquelle ausgewichen werden kann.

Die ausgezeichnete Verwertbarkeit der leicht zugänglichen Disaccharide ist mit Rücksicht auf die Verfügbarkeit des Nährsubstrats und die Wirtschaftlichkeit der PHB-Herstellung ein überraschendes und vorteilhaftes Merkmal des erfindungsgemäßen Verfahrens. In einer bevorzugten Ausführungsform wird dem Mikroorganismus Saccharose, die in industriellen Zuckerlösungen, beispielsweise Grünsirup, oder in Abfallprodukten der Zuckergewinnung, beispielsweise Rübenmelasse, enthalten ist, als Kohlenstoffquelle angeboten, wobei wiederum die Kultivierung des Stammnes Alcaligenes latus DSM Nr. 1123 mit Saccharose wegen der hohen PHB-Produktbildungsgeschwindigkeit besonders vorteilhaft ist.

Werden dem Mikroorganismus aus der Gruppe assimilierbarer Kohlenstoffverbindungen solche angeboten, die zugleich Stickstoff im Molekül haben, kann der Kohlenstoff- und Stickstoffbedarf aus derselben Quelle gedeckt werden. Als Stickstoffquelle werden von den Alcaligenes latus-Stämmen auch noch Ammoniak, Ammoniumsalze, beispielsweise Ammoniumchlorid oder Ammoniumsulfat, und Nitrate verwertet. Weiters kann der notwendige Stickstoffbedarf auch durch den Stickstoffgehalt von Industriellen und biologischen Abwässern weitgehend gedeckt werden.

Die Zusammensetzung der Nährlösung hinsichtlich ihrer weiteren mineralischen Komponenten umfaßt

3

Phosphor, beispielsweise in Form von Natriumhydrogenphosphat oder Kaliumhydrogenphosphat, Magnesium, beispielsweise als Magnesumsulfat, Calcium beispielsweise als Calciumchlorid und Eisen beispielsweise in Form von Eisen (III)-chlorid, Eisensulfat oder Eisen (III)-Ammoniumcitrat. Weitere Spurenmineralstoffe, die für das Wachstum essentiell sind, können dem Nährmedium bevorzugt in Form einer Spurenelementlösung zugegeben werden, die beispielsweise folgende Zusammensetzung hat:

| | |
|---|---|
| $ZnSO_4.7H_2O$ | 100 mg/l |
| $MnCl_2.4H_2O$ | 30 mg/l |
| $H_3BO_3$ | 300 mg/l |
| $CoCl_2.6H_2O$ | 200 mg/l |
| $CuSO_4.5H_2O$ | 10 mg/l |
| $NiCl_2.6H_2O$ | 20 mg/l |
| $NaMoO_4.2H_2O$ | 30 mg/l |

Bei der Durchführung des erfindungsgemäßen Verfahrens geht man zweckmäßigerweise so vor, daß man zunächst eine oder mehrere Vorkulturen, beispielsweise in einem flüssigen Nährmedium, herstellt und dann eine im Produktionsfermenter vorbereitete Nährlösung der angegebenen Zusammemsetzung mit der gut angewachsenen Vorkultur im Verhältnis von etwa 1:5 bis 1:15 beimpft.

Während der gesamten Dauer der Kultivierung wird durch die Ergänzung der verbrauchten Nährsubstrate dafür Sorge getragen, daß die Quellen für Kohlenstoff, Stickstoff und Phosphor, sowie für sämtliche Spurennährstoffe in der für das Bakterienwachstum optimalen Konzentration gehalten werden. Unter optimaler Nährstoffversorgung im Sinne der vorliegenden Erfindung ist die Züchtung des Mikroorganismus in einem Kulturmedium zu verstehen, das über die gesamte Dauer der Fermentierung hinweg alle Komponenten des vollständigen Nährstoffangebotes in solchen Konzentrationen enthält, daß in der Kultur unlimitierte Wachstumsbedingungen herrschen und der Mikroorganismus keinen Mangel und keine Beschränkung in der Versorgung mit wachstumsessentiellen Nährstoffen, beispielsweise mit Stickstoff oder Phosphor, erfährt.

Die optimale Versorgung mit Stickstoff für das Bakterienwachstum wird nach der Methode von Warburg beschrieben in Manometric Techniques von Umbreit W.W., Burris R.H., Stauffer J.S., Burges Publishing Company, Minneapolis, USA, 1964 am Warburg Respirator bestimmt. Für die Stämme von Alcaligenes latus ist die optimale Stickstoffversorgung, ohne für das Wachstum limitierende Bedingungen zu schaffen, in der Regel dann gegeben, wenn im Kulturmedium während der gesamten Fermentierung eine Konzentration von mindestens 200 mg Ammoniumsulfat pro Liter Kulturflüssigkeit entsprechend einer Stickstoffkonzentration von mindestens 45 m g/l vorhanden ist, wobei eine Stickstoffkonzentratlon von etwa 50 bis 700 m g/l bevorzugt ist, wobei wiederum eine Konzentratlon von 80 bis 320 m g/l besonders bevorzugt ist.

Für die optimale Phosphorversorgung ist eine Konzentration von mindestens 500 mg/l Phosphor empfehlenswert, wobei die Einhaltung einer Konzentration von 600 mg/l bis 1.2 g/l wiederum besonders bevorzugt ist. Als Kohlenstoffquelle enthält eine optimale Nährlösung beispielsweise 10 - 40 g Saccharose pro Liter Kulturmedium.

Bei der Kultivierung von Stämmen von Alcaligenes latus unter den erfindungsgemäßen Bedingungen tritt überraschenderweise keine ausgeprägte Speicherphase für PHB in Abhängigkeit von einer Nährstofflimitierung, beispielsweise durch Unterversorgung mit Stickstoff, auf, sondern es ist eine mit dem Wachstum assoziierte, äußerst effiziente PHB-Anreicherung unter für den Mikroorganismus günstigen Wachstumsbedingungen bei vollständiger Nährstoffversorgung zu beobachten. Diese wachstumsassoziierte PHB-Anreicherung zeigt sich unter anderem darin, daß der prozentuelle Gehalt an PHB in den Zellen im Verlaufe der Züchtung beispielsweise nie unter 60 Gew.-% des Zelltrockengewichts liegt.

Die wachstumsassoziierte PHB-Speicherung bei uneingeschränkter Nährstoffversorgung hat den Vorteil, daß weder die spezifische Wachstumsgeschwindigkeit noch die PHB-Produktbildungsgeschwindigkeit durch Nährstoffmangel herabgesetzt wird und daher eine größere Konzentration an Biomasse mit einer besseren PHB-Anreicherung innerhalb von wesentlich kürzeren Fermentierzeiten erhalten werden kann, als es bei den bekannten Verfahren, die unter limitierter Nährstoffversorgung arbeiten, der Fall ist.

Unter Einhaltung der erfindungsgemäßen Züchtungsbedingungen ist es beispielweise möglich, nach einer Verweilzeit im Fermenter von 34 Stunden pro Liter Kulturflüssigkeit 52.2 g trockener Biomasse mit einem PHB-Gehalt von 74 % des Zelltrockengewichts zu ernten.

Die Züchtung wird im Fermenter unter den angegebenen Bedingungen zweckmäßigerweise so lange fortgesetzt, bis die gebildete Biomasse einen PHB-Gehalt von mindestens 60 Gew.-%, vorzugsweise aber 70-80 Gew.-% des Zelltrockengewichts erreicht hat. Es ist dabei zweckmäßig, die laufende Versorgung des Nährmediums mit der Stickstoffquelle so einzurichten, daß der für die vollständige Nährstoffversorgung notwendige untere Grenzwert der Stickstoffkonzentration, also etwa eine Konzentration von 45 mg Stickstoff pro Liter Kulturflüssigkeit erst dann erreicht oder unterschritten wird, wenn der PHB-Gehalt zumindest 60 Gew.-%, vorzugsweise aber 70 - 80 Gew.-% des Zelltrockengewichts beträgt.

Der pH-Wert der Nährlösung wird durch laufende Zugabe von Pufferlösung, beispielsweise von Phosphatpufferlösung oder von wässeriger Base, beispielsweise Kalium- oder Natriumhydroxydlösung, vorteilhafterweise auf Werte zwischen 6.5 und 7.5, bevorzugt auf Werte von 6.8 bis 7.2 eingestellt.

Die Züchtung erfolgt unter aeroben Bedingungen, beispielsweise unter Sauerstoff- oder Luftzufuhr, wobei zweckmäßigerweise der Gelöstsauerstoffgehalt durch Variation der eingetragenen Luft- oder Sauerstoffmenge pro Zeiteinheit in einem Bereich zwischen 25 und 50 % des Sättigungswertes für Luft gehalten wird. Zur Förderung von ungestörtem Wachstum und PHB-Anreicherung ist es vorteilhaft, sterile Luft in das gerührte Kulturmedium einzutragen. Es ist auch möglich durch Variation der Rührerdrehzahl den Gelöstsaueratoffgehalt im gewünschten Bereich zu halten.

Die Stämme von Alcaligenes latus sind im Gegensatz zur Species Alcaligenes eutrophus überraschenderweise thermotoleranter. Diese erhöhte Thermotoleranz zeigt sich darin, daß der optimale Temperaturbereich für die Fermentierung bei 36 bis 42°C liegt, wobei wiederum eine Temperatur von 37 bis 39°C besonders bevorzugt ist. Die Züchtung in diesem Temperaturbereich hat neben der beschleunigenden Wirkung auf Wachstum und PHB-Speicherung den Vorteil, daß die Kühlkosten während der Fermentierung erheblich gesenkt werden können. Die Einsparung an Kühlkosten durch die Züchtung bei höher Temperatur in Verbindung mit den kürzeren Verweilzeiten fällt besonders ins Gewicht, da die Kühlkosten in der Regel die Hälfte der Energiekosten betragen, die beim Betrieb eines Fermentierreaktors anfallen.

Unter den angebenen Bedingungen kann die Züchtung ansatzweise, z. B. durch einmalige oder mehrmalige Zugabe von Nährlösung durchgeführt werden. Es ist aber auch möglich, den Fermenter im Wege des Zulaufverfahrens zu betrieben. Das Zulaufverfahren besteht darin, daß während der Züchtung periodisch oder kontinuierlich einzelne, mehrere oder alle Komponenten der Nährlösung der wachsenden Kultur des Mikroorganismus in steriler Form zugeführt werden, bis das Arbeitsvolumen des Fermenters erreicht ist.

Das Zulaufverfahren hat gegenüber dem ansatzweisen Verfahren den Vorteil, daß die Konzentrationen der Nährstoffe innerhalb des gewünschten Bereichs annähernd konstant gehalten werden können, bis das Fermenternutzvolumen erreicht ist.

Besonders vorteilhaft ist es aber, die Fermentierung in kontinuierlicher Arbeitsweise durchzuführen, indem der Kultur einerseits im konstanten Strom frische Nährlösung zugeführt wird und andererseits dem Fermenter eine dem Zufluß äquivalente Menge an biomassehältigem Kulturmedium entnommen wird. Durch die mit hoher Geschwindigkeit verlaufende, wachstumsassoziierte PHB-Bildung können im kontinuierlichen Betrieb bei einer PHB-Anreicherung von 70 bis 80 Gew.-% des Zelltrockengewichts noch hohe Verdünnungsraten (D) von etwa 0.4 bis 0.35 pro Stunde erreicht werden. Die Verdünnungsrate (D) ist der reziproke Wert der mittleren Verweilzeit. In allen Fällen wird darauf geachtet, daß die Ergänzung der Nährsubstrate unter sterilen Bedingungen erfolgt.

Bei der Verwendung von Kohlehydraten als Kohlenstoffquelle werden beim erfindungsgemäßen Verfahren Ertragskoeffizienten $Y_{x/s} = 0.42$ bis 0.46 erreicht, d.h. pro Gramm des eingesetzten Kohlehydrats werden 0.42 bis 0.46 Gramm Zelltrockengewicht in Form von PHB-hältiger Biomasse gewonnen Werden Melasse oder Grünsirup als Kohlenstoffquelle in der Nährlösung verwendet, ist es möglich Ertragskoeffizienten von $Y_{x/s} = 0.6$ bis 0.75 bezogen auf die Saccharosemenge, zu erreichen.

Zur Isolierung der PHB werden die Zellmassen durch Dekantieren, Filtrieren oder Zentrifugieren von der Nährlösung abgetrennt und daraus die PHB auf übliche Weise durch Extrahieren gewonnen, beispielsweise nach dem Verfahren der US-PS-4 101 533.

Die von der Biomasse befreite Nährlösung kann zur Ausnützung der zurückbleibenden Nährstoffe im Kreislauf in den Fermenter zurückgeführt und zur Erreichung der optimalen Nährstoffkonzentration mit frischer Nährlösung versetzt werden. Diese Nährlösung kann dann für weitere Züchtungen verwendet werden.

Die folgenden Beispiele erläutern die Erfindung:

**Beispiel 1:**

Vier Liter eines wässerigen Mediums I werden in einen gerührten Fermenter in sterillsierter Form eingebracht. Anschließend wird 1 Liter einer wässrigen Lösung, die 125 g/l Saccharose enthält, unter sterilen Bedingungen dazu filtriert und der Fermenterinhalt auf 36°C erwärmt. Das Medium I hat folgende Zusammensetzung pro Liter deionisierten Wassers:

| | |
|---|---|
| $Na_2HPO_4 2H_2O$: | 4.5 g/l |
| $KH_2PO_4$: | 1.5 g/l |
| $MgSO_4.7H_2O$: | 0.2 g/l |
| $(NH_4)_2SO_4$: | 0.8 g/l |
| $CaCl_2.2H_2O$ | 0.02 g/l |
| Spurenlösung | 2 ml/l |
| Fe III-$NH_4$-Citrat (17 % Fe) | 0.05 g/l |

Die Spurenlösung hat folgende Zusammensetzung:

| | |
|---|---|
| ZnSO$_4$.7H$_2$O: | 100 mg/l |
| MnCl$_2$.4H$_2$O: | 30 mg/l |
| H$_3$BO$_3$: | 300 mg/l |
| CoCl$_2$.6H$_2$O: | 200 mg/l |
| CuSO$_4$.5H$_2$O: | 10 mg/l |
| NiCl$_2$.6H$_2$O: | 20 mg/l |
| NaMoO$_4$.2H$_2$O: | 30 mg/l |

Das Medium wird mit einer Vorkultur von Alcaligenes latus Stamm DSM 1124 beimpft und die Kultivierung des Mikroorganismus wird unter aeroben Bedingungen bei einem Gelöstsauerstoffpartialdruck zwischen 25 und 35 % des Sättigungswertes für Luft fortgesetzt. Während der Fermentation wird der pH-Wert der Nährlösung durch automatische Titration mit einer 10-%-igen sterilen wässerigen NaOH-Lösung bei 7.0 konstant gehalten. Im weiteren Verlauf der Fermentierung wird durch mehrmaligen Zusatz von Saccharose und Ammoniumsulfat dafür gesorgt, daß sich die Konzentration dieser Substrate nie unter 10 % des Anfangswerts senkt. Nach einer Verweilzeit im Fermenter von 39 Stunden haben sich 47,6 g/l trockene Biomasse mit einem PHB-Gehalt von 69 Gew.-% gebildet. Der Ertragskoeffizient $Y_{x/s}$ (g Zelltrockengewicht/g eingesetzter Saccharose) = 0.45.

**Beispiel 2:**

Ein Mikroorganismus vom Stamm Alcaligenes latus DSM 1123 wird unter den in Beispiel 1 angegebenen Verfahrensbedingungen bei einer Temperatur von 38°C gezüchtet. Nach einer Verweilzeit im Fermenter von 34 Stunden haben sich 51.3 g/l trockene Biomasse mit einem PHB-Gehalt von 73,8 Gew.-% gebildet. Der Ertragskoeffizient $Y_{x/s}$ = 0.46.

**Beispiel 3:**

Ein Mikroorganismus vom Stamm Alcaligenes latus DSM 1122 wird unter den in Beispiel 1 angegebenen Verfahrensbedingungen bei einer Temperatur von 36°C gezüchtet. Nach einer Verweilzeit im Ferm enter von 37 Stunden haben sich 48.1 g/l trockene Biomasse mit einem PHB-Gehalt von 70.2 Gew.-% gebildet. Der Ertragskoeffizient $Y_{x/s}$ = 0.45.

**Beispiel 4:**

20 Liter eines Kulturmediums, das eine Anfangskonzentration von 2.2 g/l an (NH$_4$)$_2$SO$_4$ und 22 g/l an Saccharose als Kohlenstoffquelle enthält und sonst die gleiche Zusammensetzung wie das in Beispiel 1 angegegebene Medium I aufweist, werden in einem sterilen Fermenter mit einem Arbeitsvolumen von 100 l sterilfiltriert vorgelegt. Anschließend wird der Fermenter mit 2.5 l einer gut angewachsenen Vorkultur von Alcaligenes latus vom Stamm DSM 1123 beimpft. Durch Variation von Rührerdrehzahl und Belüftungsrate wird der Gelöstsauerstoffgehalt auf eine Konzentration von 25 bis 35 % des Sättigungswertes für Luft eingestellt und während der Fermentierung wird eine Temperatur von 37°C eingehalten. Der pH-Wert der Nährlösung wird durch automatische Titration mit steriler 10-%-iger wässeriger NaOH-Lösung auf 7.0 gehalten.

Im weiteren Verlauf der Fermentierung werden jeweils zum Zeitpunkt der Abnahme der Konzentration an (NH$_4$)$_2$SO$_4$ auf etwa 0.3 g/l, Medium I, Saccharoselösung mit einer Konzentration von etwa 500 g/l und (NH$_4$)$_2$SO$_4$-Lösung mit einer Konzentration von 200 g/l unter sterilen Bedingungen stoßweise in Abhängigkeit vom jeweils Fermenter vorhandenen Volumen der Kulturbrühe in solchen Mengen zugesetzt, daß die Konzentrationen der einzelnen Nährstoffkomponenten immer über den limitierenden Konzentrationen gehalten werden. Insgesamt werden während der Züchtung bis zum Erreichen des Fermenternutzvolumens 1327 g (NH$_4$)$_2$SO$_4$, entsprechend einer Stickstoffmenge von 281.5 g, zugesetzt. Nach 34 Stunden ist mit einem Volum en von 100 l Kulturflüssigkeit das Nutzvolumen des Fermenters ausgeschöpft und man erntet 52,2 g/l trockene Biomasse mit einem PHB-Gehalt von 74 Gew.-%, was einer Gesamtmenge von 5220 g trockener Biomasse, die 3863 g PHB enthält. Der Ertragskoeffizient $Y_{x/s}$ = 0.46.

90 % des Fermenterinhaltes werden zur Aufarbeitung der Biomasse und Extraktion der PHB aus dem Fermenter abgezogen. Der restliche Inhalt des Fermenters dient als Vorkultur für einen neuen Arbeitszyklus. Nach 27 Stunden ist das Nutzvolum en des Fermenters wiederum erreicht und man erhält 52.3 g/l trockene Biomasse mit einem PHB-Gehalt von 74.4 Gew.-%. Der Ertragskoeffizient ist gegenüber der ersten Fermentierung unverändert hoch.

Innerhalb von weiteren 5 Arbeitszyklen bleiben die Ergebnisse bei der Fermentierung sowohl hinsichtlich der erreichten Biomassekonzentration und des prozentuellen PHB-Anteils an der Biomasse als auch hinsichtlich des Ertragskoeffizienten unverändert.

**Beispiel 5:**

20 Liter eines Kulturmediums, das eine Anfangskonzentration von 15 g/l Saccharose als einzige Kohlenstoffquelle enthält und sonst die gleiche Zusammensetzung wie das Medium I aufweist werden wie in Beispiel 4 unter den dort angegebenen Bedingungen beimpft.

Im weiteren Verlaufe der Züchtung wird das Zulaufverfahren kontinuierlich durchgeführt, in dem die Konzentrationen der Nährsubstrate durch einen den Verbrauchsgeschwindigkeiten und der Verdünnung angepaßten Zulauf mit frischen Nährlösungen konstant gehalten werden. Die frischen Nährlösungen bestehen aus Saccharoselösung mit einer Konzentration von 300 g/l, $(NH_4)SO_4$-Lösung mit einer Konzentration von 200 g/l und frischem Medium I.

Nach 28 Stunden ist unter diesen kontinuierlichen Zulaufbedingungen das maximale Arbeitsvolumen des Fermenters von 100 l ausgeschöpft. Die Kulturbrühe enthält zu diesem Zeitpunkt 46 g/l an trockener Biomasse mit einem PHB-Gehalt von 74 Gew.-%. Der Ertragskoeffizient $Y_{x/s} = 0.46$.

Wie in Beispiel 4 werden 90 % des Fermenterinhaltes zur Aufarbeitung der Biomasse aus dem Fermenter abgezogen und der restliche Inhalt des Fermenter dient als Inoculum für die neuerliche Aufzucht nach dem beschriebenen, kontinuierlichen Zulaufverfahren. In fünf weiteren Arbeitszyklen wurden hinsichtlich der Biomasse, des PHB-Gehalts und des Ertragskoeffizienten die gleichen Ergebnisse wie im ersten Zyklus erhalten.

**Beispiel 6:**

20 Liter eines Kulturmediums, das eine Anfangskonzentration von 1.2 g/l $(NH_4)_2SO_4$ als Stickstoffquelle und 8 g Glucose als Kohlenstoffquelle enthält und sonst die gleiche Konzentration wie das in Beispiel 1 angegebene Medium aufweist, werden in einem sterilen, gerührten Fermenter mit einem Nutzvolumen von 100 l sterilfiltriert vorgelegt. Anschließend wird der Fermenter mit 2l einer gut angewachsenen Vorkultur von Alcaligenes latus DSM 1122 beimpft. Der Gelöstsauerstoffgehalt wird durch Variation der Rührerdrehzahl und der Belüftungsrate auf einen Konzentrationsbereich von 38 bis 42 % des Sättigungswerts für Luft eingestellt. Während der Fermentierung wird eine Temperatur von 39°C eingehalten und der pH-Wert der Nährlösung wird durch automatische Titration mit steriler 10-%-iger wässeriger NaOH-Lösung auf 7.0 gehalten.

Im weiteren Verlauf der Züchtung werden die Konzentrationen von $(NH_4)_2SO_4$ und Glucose durch kontinuerlichen Zulauf von frischen Nährmedium I und keimfreien Lösungen von $(NH_4)_2SO_4$ und Glucose konstant gehalten.

Nach 23 Stunden Betriebszeit ist unter den angegebenen Bedingungen das maximale Arbeitsvolumen des Reaktors erreicht. Die Nährlösung enthält zu diesem Zeitpunkt 39 g/l an trockener Biomasse mit einem PHB-Gehalt von 74 Gew.-%. Der Ertragskoeffizient $Y_{x/s} = 0.42$.

Der Ablauf der Züchtung kann wie in den Beispielen 4 und 5 in mehreren Arbeitszyklen mit gleichbleibenden Ergebnissen wiederholt werden.

**Beispiel 7:**

Die Züchtung wird nach dem in Beispiel 4 angegebenen diskontinuierlichen Zulaufverfahren durchgeführt, wobei als Kohlenstoffquelle Grünsirup mit einem Saccharosegehalt von 59 Gew.-% verwendet wird. Nach 31 Stunden erhält man eine Biomassekonzentration von 47 g/l mit einem PHB-Gehalt von 71 Gew.-% des Zelltrockengewichts. Der Ertragskoeffizient $Y_{x/s} = 0.62$, bezogen auf die im Grünsirup enthaltene Saccharosemenge.

**Beispiel 8:**

Die Züchtung wird nach dem in Beispiel 5 angegebenen kontinuierlichen Zulaufverfahren durchgeführt, wobei als Kohlenstoffquelle Grünsirup mit einem Saccharosegehalt von 59 Gew.-% dient. Nach 30 Stunden erhält man im Fermenter eine Biomassekonzentration von 49 g/l mit einem PHB-Gehalt von 72 % des Zelltrockengewichts. Der Ertragskoeffizient $Y_{x/s} = 0.62$, bezogen auf die im Grünsirup enthaltene Saccharosemenge.

**Beispiel 9:**

Die Züchtung wird nach dem in Beispiel 4 angegebenen diskontinuierlichen Zulaufverfahren durchgeführt, wobei als Kohlenstoffquelle Rübenmelasse mit einem Saccharosegehalt von 44 Gew.-% Verwendung findet. Nach 33 Stunden ist unter den angegebenen Bedingungen das maximale Arbeitsvolumen des Fermenters erreicht. Die Kulturbrühe enthält zu diesen Zeitpunkt 44 g/l trockener Biomasse mit einem PHB-Gehalt von 74 Gew.-%. Der Ertragskoeffizient $Y_{x/s}$ = 0.72, bezogen auf die in der Rübenmelasse enthaltene Saccharosemenge.

**Beispiel 10:**

Die Züchtung wird nach dem in Beispiel 5 angegebenen kontinuierlichem Zulaufverfahren durchgeführt, wobei als Kohlenstoffquelle Rübenmelasse mit einem Saccharosegehalt von 44 Gew.-% Verwendung findet. Unter den angegebenen kontinuierlichen Zulaufbedingungen erhält man nach 32 Stunden 47 g/l an trockener Biomasse mit einem PHB-Gehalt von 71 Gew.-%. Der Ertragskoeffizient $Y_{x/s}$ = 0.72, bezogen auf die in der Rübenmelasse enthaltene Saccharosemenge.

**Beispiel 11:**

In einem sterilen Fermenter mit einem Arbeitsvolumen von 15 Litern werden 10 l eines Kulturmediums vorgelegt, das eine Anfangkonzentration von 1.5 g/l $(NH_4)_2SO_4$ und 15 g/l Saccharose als Kohlenstoffquelle enthält und sonst die gleiche Zusammensetzung wie das Beispiel 1 angegebene Medium I aufweist, und mit 1 l einer gut angewachsenen Vorkultur von Alcaligenes latus Stamm DSM 1123 beimpft. Der Gelöstsauerstoffgehalt wird durch Variation der Rührerdrehzahl und Belüftungsrate im Beriech bei 30 % des Sättigungswertes für Luft gehalten. Während der Fermentierung wird eine Temperatur von 37°C eingehalten und der pH-Wert durch automatische Titration mit steriler 10%-iger wässeriger NaOH-Lösung auf 7.0 eingestellt.

In der weiteren Folge der Fermentierung werden analog zu Beispiel 5 durch steten Zulauf von $(NH_4)_2SO_4$, Saccharose und Medium I die verbrauchten Nährsubstrate ergänzt.

Mit Erreichen des maximalen Arbeitsvolumens von 15 Liter wird ein kontinuierliche Arbeitsweise eingerichtet, in dem der Kultur einerselts in konstantem Strom frische Nährlösungen zugeführt wird und andererseits dem Fermenter eine dem Zufluß äquivalente, biomassehältige Menge an Nährlöeung entnommen wird, deren Biomasse durch Zentrifugation gewonnen wird. Die frisch zugeführte Nährlösung enthält 40 g/l Saccharose, 4.6 g/l $(NH_4)_2SO_4$ und entspricht in ihrer übrigen Zusammensetzung dem Medium I.

Nach einiger Zeit wird ein stabiler Gleichgewichtszustand erreicht, bei dem die Kulturbrühe im Fermenter eine Konzentration von 16.5 g/l bei einem PHB-Gehalt von 71 Gew.-% des Zelltrockengewichts aufweist. Der Ablauf aus dem Fermenter enthält eine Restkonzentration von 0.45 g/l an $(NH_4)_2SO_4$ und von 4.1 g/l an Saccharose und kann nach Abtrennung der Biomasse und Ergänzung mit frischem Nährsubstraten als Nährlösung wieder in den Fermenter zurückgeführt werden.

Im Gleichgewichtszustand werden dem Fermenter 6 l/h an frischem Nährmedium zugeführt, was einer Verdünnungsrate von D = 0.4. $h^{-1}$ entspricht. Der Ertragskoeffizient $Y_{x/s}$ = 0.46. Die kontinuierliche Arbeitswiese kann über mehrere Wochen aufrecht erhalten werden, ohne daß sich hinsichtlich des Ertragskoeffizienten oder der Zusammnensetzung der Biomasse Änderungen ergeben. In der nachfolgenden Tabelle I sind die Betriebsbedingungen und die Ergebnisse der kontinuierlichen Herstellung von PHB mit Alcaligenes latus Stamm DSM 1123 zusammengestellt.

**Tabelle 1:**
Betriebsbedingungen und Ergebnisse zur kontinuierlichen Herstellung von PHB mit Alcaligenes latus Stamm DSM 1123

| | |
|---|---|
| Arbeitsvolumen | 15 l |
| Rührung | Querblattrührer |
| Temperatur | 37°C |
| pH-Wert | 7,0 |
| $O_2$-Konzentration (% der Luftsättigung) | 30 % |
| Verdünnungsrate D | 0.4 |
| Kohlenstoffquelle | Saccharose |
| Saccharosekonzentration im Zulauf | 40 g/l |
| Stickstoffquelle | $(NH_4)_2SO_4$ |
| Konzentration $(NH_4)_2SO_4$ im Zulauf | 4,6 g/l |
| Restkonzentration Saccharose | 4,1 g/l |
| Restkonzentration $(NH_4)_2SO_4$ | 0.45 g/l |
| Saccharoseverbrauch | 35.9 g/l |
| $(NH_4)_2SO_4$-Verbrauch | 4.15 g/l |
| Biomassekonzentration | 16.5 g/l |
| PHB-Gehalt der Biomasse | 71 % |
| Produktivität Biomasse | 99.1 g/h |
| Produktivität PHB | 70.4 g/h |
| $YX$/Saccharose | 0.46 |
| $YX$/$(NH_4)_2SO_4$ | 3,98 |

## Patentansprüche

1. Verfahren zur biotechnologischen Herstellung von Poly-D(-)-3-hydroxybuttersäure ddurch aerobe Kultivierung eines Mikroorganismus der Gattung Alcaligenes in einem wässerigen Nährmedium, das Quellen für assimilierbaren Kohlenstoff, Stickstoff und Phosphor, sowie das für das Wachstum des Mikroorganismus erforderliche Angebot an organischen und anorganischen Spurennährstoffen enthält, dadurch gekennzeichnet, daß man einen Stamm von Alcaligenes latus bei vollständiger und für das Wachstum des Mikroorganismus optimaler Nährstoffversorgung unter unlimitierten Wachstumsbedingungen im Temperaturbereich von 36 bis 42°C bei einem Gelöstsauerstoffgehalt von 25 bis 50 % des Sättigungswertes für Luft und einem pH-Wert von 6.5 bis 7.5 unter sterilen Bedingungen züchtet und aus der dabei erhaltenen Biomasse die PHB auf übliche Weise durch Extraktion gewinnt.

2. Verfahren nach Anspruch 1, dadurch gekennzeichnet, daß die Stämme von Alcaligenes latus DSM Nr. 1122, DSM Nr. 1123 oder DSM Nr. 1124 eingesetzt werden.

3. Verfahren nach den Ansprüchen 1 und 2, dadurch gekennzeichnet, daß man als Kohlenstoffquelle Saccharose verwendet.

4. Verfahren nach den Ansprüchen 1 bis 3, dadurch gekennzeichnet, daß man den Stamm Alcaligenes latus DSM Nr. 1123 mit Saccharose als einziger Kohlenstoffquelle züchtet.

5. Verfahren nach den Ansprüchen 1 bis 4 dadurch gekennzeichnet, daß man die Züchtung in einem Nährmedium vornimmt, welches pro Liter Kulturflüssigkeit 10 bis 40 g Saccharose als Kohlenstoffquelle enthält.

6. Verfahren nach den Ansprüchen 1 bis 5, dadurch gekennzeichnet, daß man die Züchtung in einem Nährmedium vornimmt, welches während der gesamten Dauer der Ferm entierung pro Liter Kulturflüssigkeit mindestens 45 mg Stickstoff enthält.

7. Verfahren nach den Ansprüchen 1 bis 6, dadurch gekennzeichnet, daß man die Züchtung in einem Nährmedium vornimmt, welches pro Liter Kulturflüssigkeit 80 bis 320 mg Stickstoff enthält.

8. Verfahren nach den Ansprüchen 1 bis 7, dadurch gekennzeichnet, daß man die Züchtung in einem Nährmedium vornimmt, welches pro Liter Kulturflüssigkeit mindestens 500 mg Phosphor enthält.

9. Verfahren nach den Ansprüchen 1 bis 8, dadurch gekennzeichnet, daß man die Züchtung in einem Nährmedium vornimmt, welches pro Liter Kulturflüssigkeit von 600 mg bis 1,2 g Phosphor enthält.

10. Verfahren nach den Ansprüchen 1 bis 9, dadurch gekennzeichnet, daß man die Züchtung so lange fortsetzt, bis die gebildete Biomasse eine PHB-Konzentration von 70 bis 80 Gew.-% des Zelltrockengewichts erreicht hat.

11. Verfahren nach den Ansprüchen 1 bis 10, dadurch gekennzeichnet, daß man die Züchtung bei einem pH-Wert von 6.8 bis 7.2 durchführt.

12. Verfahren nach den Ansprüchen 1 bis 11, dadurch gekennzeichnet, daß man die Züchtung bei einer Temperatur von 37 bis 39°C durchführt.

13. Verfahren nach den Ansprüchen 1 bis 12, dadurch gekennzeichnet, daß man die Züchtung im Wege des Zulaufverfahrens durchführt.

14. Verfahren nach den Ansprüchen 1 bis 12, dadurch gekennzeichnet, daß man die Züchtung kontinuierlich durchführt.

15. Verfahren nach den Ansprüchen 1 bis 14, dadurch gekennzeichnet, daß man die Nährlösung nach Abtrennung der Biomasse im Kreislauf in den Fermenter zurückführt und für weitere Züchtungen mit frischer Nährlösung ergänzt.

**Claims**

1. Process for the biotechnological preparation of poly-D(-)-3-hydroxybutyric acid by aerobic cultivation of a microorganism of the genus Alcaligenes in an aqueous nutrient medium which contains sources of assimilable carbon, nitrogen and phosphorus, as well as the amount of organic and inorganic trace nutrients necessary for the growth of the microorganism, characterized in that a strain of Alcaligenes latus is cultured with a supply of nutrients which is complete and optimal for the growth of the microorganism, under unlimited growth conditions, in the temperature range 36 to 42°C, with a dissolved oxygen content of 25 to 50 % of the saturation value for air, and at a pH of 6.5 to 7.5 under sterile conditions, and the PHB is isolated in a customary manner by extraction from the biomass which is obtained thereby.

2. Process according to Claim 1, characterized in that the strains DSM No. 1122, DSM No. 1123 or DSM No. 1124 of Alcaligenes latus are used.

3. Process according to Claims 1 and 2, characterized in that sucrose is used as carbon source.

4. Process according to Claims 1 to 3, characterized in that the strain Alcaligenes latus DSM No. 1123 is cultured with sucrose as the only carbon source.

5. Process according to Claims 1 to 4, characterized in that the culturing is carried out in a nutrient medium which contains 10 to 40 g of sucrose as carbon source per litre of culture liquid.

6. Process according to Claims 1 to 5, characterized in that the culturing is carried out in a nutrient medium which, throughout the duration of the fermentation, contains at least 45 mg of nitrogen per litre of culture liquid.

7. Process according to Claims 1 to 6, characterized in that the culturing is carried out in a nutrient medium which contains 80 to 320 mg of nitrogen per litre of culture liquid.

8. Process according to Claims 1 to 7, characterized in that the culturing is carried out in a nutrient medium which contains at least 500 mg of phosphorus per litre of culture liquid.

9. Process according to Claims 1 to 8, characterized in that the culturing is carried out in a nutrient medium which contains from 600 mg to 1.2 g of phosphorus per litre of culture liquid.

10. Process according to Claims 1 to 9, characterized in that the culturing is continued until the biomass which has formed has reached a PHB concentration of 70 to 80 % by weight of the dry weight of the cells.

11. Process according to Claims 1 to 10, characterized in that the culturing is carried out at a pH of 6,8 to 7.2.

12. Process according to Claims 1 to 11, characterized in that the culturing is carried out at a temperature of 37 to 39°C.

13. Process according to Claims 1 to 12, characterized in that the way the culturing is carried out is by the feed process.

14. Process according to Claims 1 to 12, characterized in that the culturing is carried out continuously,

15. Process according to Claims 1 to 14, characterized in that the nutrient solution is, after removal of the biomass, recycled to the fermenter and replenished with fresh nutrient solution for further culturing.

**Revendications**

1. Procédé de préparation biotechnologique d'acide poly-D(-)-3-hydroxybutyrique par culture aérobie d'un moicro-organisme du genre Alcaligenes dans un milieu nutritif aqueux qui contient des sources de carbone, azote et phosphore assimilables, ainsi que l'apport en substance nutritives organiques et inorganiques a l'état de traces, nécessaire pour la croissance du micro-organisme, caractérisé en ce qu'on cultive dans des conditions stériles une souche d'Alcaligenes latus en presence d'une alimentation de substance nutritive complète et optimale pour la croissance du micro-organisme, dans des conditions de croissance non limitées et dans l'intervalle de températures de 36°C à 42°C, en présence d'une teneur en oxygène dissous de 25 à 50 % de la valeur de saturation pour l'air et d'une valeur de pH de 6,5 à 7,5, et on récupère le PHB de manière courante par extraction à partir de la biomasse ainsi obtenue.

2. Procédé selon la Revendication 1, caractérisé en ce qu'on introduit les souches d'Alcaligenes latus DSM N°1122, DSM N° 1123 ou DSM N°1124.

3. Procédé selon les Revendications 1 et 2, caractérisé en ce qu'on utilise comme source de carbone, le saccharose.

4. Procédé selon les Revendication 1 à 3, caractérisé en ce qu'on cultive la souche Alcaligenes latus DSM N°1123 avec le saccharose comme seule source de carbone.

5. Procédé selon les Revendications 1 à 4, caractérisé en ce que l'on procède à la culture dans un milieu nutritif qui contient par litre de liquide de culture, 10 à 40 g de saccharose comme source de carbone.

6. Procédé selon les Revendications 1 à 5, caractérisé en ce que l'on procède à la culture dans un milieu nutritif qui contient pendant toute la durée de la fermentation par litre de liquide de culture, au moins 45 g d'azote.

7. Procédé selon les Revendications 1 à 6, caractérisé en ce que l'on entreprend la culture dans un milieu nutritif qui contient par litre de liquide de culture, 80 à 320 mg d'azote.

8. Procédé selon les Revendications 1 à 7, caractérisé en ce que l'on entreprend la culture dans un milieu nutritif qui contient par litre de liquide de culture, au moins 500 mg de phosphore.

9. Procédé selon les Revendications 1 à 8, caractérisé en ce que l'on entreprend la culture dans un milieu nutritif qui contient par litre de liquide de culture, de 600 mg à 1,2 g de phosphore.

10. Procédé selon les Revendications 1 à 9, caractérisé en ce que l'on poursuit la culture jusqu'à ce que la biomasse formée ait atteint une concentration en PHB de 70 à 80 % en poids par rapport au poids sec des cellules.

11. Procédé selon les Revendications 1 à 10, caractérisé en ce que l'on effectue la culture a une valeur de pH de 6,8 à 7,2.

12. Procédé selon les Revendications 1 à 11, caractérisé en ce que l'on effectue la culture à une température de 37 à 39°C.

13. Procédé selon les Revendications 1 à 12, caractérisé en ce que l'on effectue la culture suivant le mode opératoire à apports d'alimentation ajustables.

14. Procédé selon les Revendications 1 à 12, caractérisé en ce que l'on effectue la culture en continu.

15. Procédé selon les Revendications 1 à 14, caractérisé en ce qu'après avoir séparé la biomasse, on recycle la solution nutritive dans le fermenteur et on la complate avec de la solution nutritive fraîche pour d'autres cultures.